# FASCICULE DE BREVET EUROPEEN

(11) **EP 1 073 884 B1**
(45) Date de publication et mention de la délivrance du brevet: **24.07.2002**
(21) Numéro de dépôt: 99915812.4
(22) Date de dépôt: 22.04.1999
(51) Int. Cl.: G01F 1/48, A61M 1/00, G01L 19/00, G01F 1/34

(54) **DISPOSITIF DE MESURE DE DEBIT**
DURCHFLUSSMESSVORRICHTUNG
FLOW RATE MEASURING DEVICE

(30) Priorité: 24.04.1998 FR 9805189
(43) Date de publication de la demande: 07.02.2001
(73) Titulaire: CORNEAL INDUSTRIE, 74370 Pringy (FR)
(72) Inventeur: BOS, Gilles, F-74330 La Balme de Sillingy (FR); DUCOURNAU, Didier, F-44000 Nantes (FR); VITALLY, Guy, F-74370 Villaz (FR)
(74) Mandataire: Dronne, Guy
(86) Numéro de dépôt international: FR9900956
(87) Numéro de publication internationale: WO9956090

(56) Documents cités:
- EP-A- 0 027 402
- DE-A- 4 308 313
- US-A- 5 410 916

## Description

La présente invention a pour objet un dispositif de mesure du débit d'un liquide circulant dans une tubulure vers ou hors du corps humain.

De façon plus précise, l'invention concerne un dispositif qui permet de mesurer le débit d'un liquide aqueux qui circule dans une tubulure raccordée à une partie du corps humain et qui est mis en circulation à l'aide d'une pompe d'aspiration.

Un tel problème se présente notamment dans le cas de la mise en oeuvre de la technique chirurgicale dite vitrectomie. Cette technique de chirurgie oculaire consiste à réaliser l'ablation de l'humeur vitrée par fragmentation de celle-ci à l'aide d'un outil pneumatique à guillotine lors d'une intervention chirurgicale sur la partie postérieure de l'oeil. Les fragments de l'humeur vitrée doivent être extraits de l'oeil à l'aide d'une canule. Simultanément, à l'aide d'une autre canule, on injecte dans l'oeil un liquide physiologique pour remplacer temporairement l'humeur vitrée extraite. L'injection est réalisée par un simple effet de gravité afin de conserver le volume naturel de l'oeil en maintenant le liquide remplissant l'oeil à une pression constante.

Dans une telle opération, il est extrêmement important de maîtriser le volume de fluide extrait quelle que soit la dépression à générer en bout de l'outil afin de ne pas aspirer et donc endommager les tissus environnants. Dans le cas de cette intervention, cet aspect est d'autant plus déterminant que la chirurgie est réalisée à proximité de la rétine. On comprend en effet qu'en cas de dépressurisation de l'oeil, on risquerait de produire un décollement de la rétine.

Les techniques actuelles ne permettent de maîtriser que la dépression au niveau de la mesure et donc le volume aspiré est variable selon la configuration matérielle de la tubulure de l'outil et la présence éventuelle de pseudo-occlusions résultant de l'aspiration de l'humeur vitrée fragmentée. De plus, dans le cas de très faibles débits, il faut générer une très faible aspiration. Une très faible variation de la dépression pourrait avoir des conséquences très dommageables pour le patient.

Le document DE 43 08 313 décrit, dans le domaine médical, un système de mesure de débit dans une conduite qui comprend deux volumes de prise de pression reliés à la conduite et reliés entre eux par une tubulure. Cependant, ce système résouds un problème différent.

Un objet de la présente invention est de fournir un dispositif de mesure directe du débit du liquide circulant dans la tubulure raccordée d'une part à la canule placée dans l'oeil du patient et d'autre part à la pompe d'aspiration avec la précision requise, et de plus sensiblement en temps réel, c'est-à-dire sans effet d'hystérésis.

Pour atteindre ce but, selon l'invention, le dispositif de mesure du débit d'un liquide circulant dans une tubulure et extrait ou introduit dans le corps humain comprenant deux moyens de mesure de pression, chaque moyen de mesure de pression comportant une première extrémité raccordée respectivement à une première et une deuxième portion de ladite tubulure, et une deuxième extrémité de raccordement et un élément de conduite dont les extrémités sont respectivement raccordées aux deuxièmes extrémités de raccordement desdits capteurs, caractérisé en ce que :
ledit élément de conduite est sensiblement rigide et présente un diamètre interne D sensiblement égal à celui des portions de tubulure pour produire un écoulement laminaire dudit liquide ;
en ce que chaque moyen de mesure de pression comprend en outre :
   - des moyens formant tubulure présentant un alésage axial dont la face interne de la paroi constitue sensiblement une solution de continuité de la tubulure et s'étendant entre les deux extrémités de raccordement, lesdits moyens comportant un orifice traversant la paroi et débouchant dans la face interne, et
   - un capteur de pression pour mesurer la pression dudit liquide dans la tubulure, ledit capteur présentant une surface soumise à la pression dudit liquide, au moins une partie dudit capteur étant montée de manière étanche dans ledit orifice, de telle manière que ladite surface sensible constitue sensiblement une solution de continuité de la face interne de la paroi des moyens formant tubulure.
et en ce que ledit dispositif comprend en outre des moyens de traitement des deux pressions mesurées pour en déduire un débit.

On comprend que, grâce au dispositif de mesure selon l'invention, on obtient tout d'abord une mesure de pression très précise et sans hystérésis à l'aide des deux moyens de mesure de pression et que, compte tenu du fait que la portion de conduite entre ces deux capteurs présente des caractéristiques permettant d'assurer un écoulement laminaire du liquide dans celle-ci, on peut déduire de ces deux mesures de pression une mesure de débit basée sur la relation qui existe entre la perte de charge entre les deux points de mesure et la différence de pression en ces deux points.

D'autres caractéristiques et avantages de l'invention apparaîtront mieux à la lecture de la description qui suit d'un mode de réalisation de l'invention.

La description se réfère aux figures annexés sur lesquelles :
- la figure 1 est une vue en coupe longitudinale de l'ensemble du dispositif de mesure de débit ;
- la figure 2 est une vue en coupe longitudinale d'un capteur de pression ; et
- la figure 3 est une vue du capteur de pression en coupe selon la ligne III-III de la figure 2.

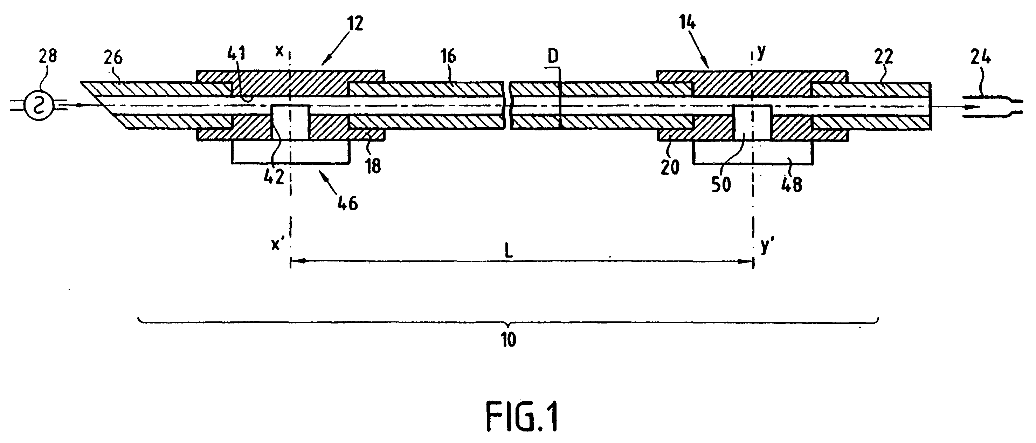

En se référant tout d'abord à la figure 1, on va décrire l'ensemble du dispositif de mesure de débit dans la tubulure. Le dispositif de mesure proprement dit référencé 10 est constitué essentiellement par deux dispositifs de mesure de pression 12 et 14 sensiblement identiques et qui seront décrits en détail ultérieurement, entre lesquels est raccordé un élément de conduite 16. Plus précisément, les extrémités de la conduite 16 sont fixées dans des extrémités de raccordement 18 et 20 des capteurs de pression 12 et 14. Le dispositif de mesure de débit 10 est raccordé aux deux portions de la tubulure dans lesquelles circule le liquide dont on veut mesurer le débit. Sur la figure 1, on a représenté une partie de la première portion de tubulure 22 qui est par exemple raccordée à une canule 24 de vitrectomie et une deuxième portion de tubulure 26 raccordée à une pompe d'aspiration 28. La portion de conduite 16 est de préférence réalisée en silicone et sa longueur L ou plus précisément la longueur entre les axes XX' et YY' des deux capteurs de pression est comprise entre 1,5 m et 2,5 m et de préférence égale à 2 m. Le diamètre intérieur D de l'élément de conduite 16 est déterminé de telle manière que l'on ait un écoulement laminaire dans cette portion de tube et donc un écoulement laminaire entre les deux capteurs de pression. Ce diamètre D est par exemple égal à 1,5 mm.

Pour assurer l'écoulement laminaire, il est nécessaire que le nombre de Raynolds soit inférieur à 2 000. Cette condition impose que le diamètre hydraulique de la portion de conduite 16 soit supérieur à 0,53 mm. Il est également nécessaire, pour assurer un écoulement laminaire, que l'élément de conduite 16 ne présente pas de faibles rayons de courbure notamment dus à sa déformation sous son propre poids. Il est donc nécessaire que l'élément de conduite présente une certaine rigidité. Pour cela, on peut utiliser une conduite en silicone dont la paroi a une épaisseur de l'ordre de 0,8 mm. En outre, pour pouvoir mesurer le débit, il est nécessaire que la perte de charge créée par l'élément de conduite entre les deux capteurs soit suffisante pour pouvoir être mesurée avec précision. Cette perte de charge peut être estimée à au minimum 1 mm Hg. Si l'on considère des débits classiquement utilisés de 1 à 50 cm³/min, cette condition impose que Cela est vrai si la rugosité de la face interne de la conduit est négligeable, ce qui est le cas, et si la portion de conduite est relativement rigide pour le pas présenter une courbure importante lors de l'utilisation du dispositif, ainsi qu'on l'a déjà expliqué.

De façon standard, les diamètres de tubulure 16 ont de 1 à 3 mm de diamètre interne. Si l'on utilise un diamètre interne de 1,5 mm, on obtient une longueur L égale à 2 m. Ces fourchettes correspondent au cas où le liquide en circulation dans la tubulure présente une viscosité proche de l'eau.

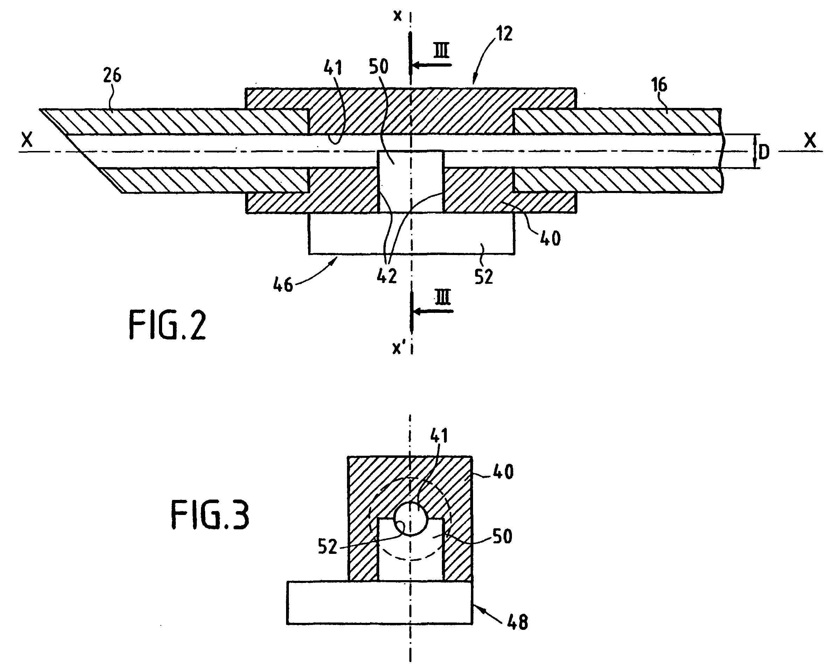

En se référant maintenant plus particulièrement aux figures 2 et 3, on va décrire un mode préféré de réalisation du dispositif de mesure de pression, par exemple du dispositif 12. Celui-ci est constitué par un corps de forme sensiblement cylindrique 40 percé d'un alésage axial 41 de diamètre D. De préférence le corps 40 est constitué en un matériau biocompatible tel que du PMMA. Le corps 40 est percé d'un orifice 42 dont l'axe XX' est disposé selon un diamètre de l'évidement axial 41 et débouche dans celui-ci. L'évidement 42 sert à la mise en place d'un capteur de pression 46 qui comporte une partie de connexion et de traitement électrique 48 et une extension 50 pénétrant dans l'orifice 42 et se terminant par une surface sensible 52. Cette surface sensible 52 a une forme semi-cylindrique qui constitue ainsi une surface de continuité avec la paroi interne de l'alésage axial 41 lorsque le capteur est mis en place dans l'orifice 42. L'intérêt de cette solution est que l'on évite au niveau de la surface sensible 52 du capteur la présence d'une cavité par rapport à l'alésage axial 42 dans laquelle pourrait venir s'accumuler du gaz. En effet, ce gaz étant compressible, il risquerait d'altérer la mesure de pression ou au moins d'introduire un phénomène d'hystérésis lors de cette mesure.

Si l'on revient à l'ensemble du dispositif 10 de mesure de débit, on comprend que, en mesurant avec précision à l'aide des dispositifs de mesure 12 et 14, la pression à leur niveau respectif entre ces deux capteurs, on peut faire une mesure de pression différentielle qui dépend à la fois du débit du liquide dans la tubulure et de la perte de charge entre les deux points de mesure. Cette perte de charge étant parfaitement déterminée par les caractéristiques géométriques de la portion de conduite 16 et la qualité de l'état de surface de la paroi interne de la tubulure, on peut en déduire le débit effectif circulant dans l'élément de conduite 16 et donc dans la tubulure 22, 26.

De préférence, le volume mort limité par la surface sensible du capteur et par la surface sur laquelle est inscrite la face interne de la paroi des moyens formant tubulure est inférieur à 10 mm³.

## Revendications

1. Dispositif de mesure du débit d'un liquide circulant dans une tubulure et extrait ou introduit dans le corps humain comprenant deux moyens de mesure de pression, chaque moyen de mesure de pression comportant une première extrémité raccordée respectivement à une première et une deuxième portion de ladite tubulure, et une deuxième extrémité de raccordement et un élément de conduite dont les extrémités sont respectivement raccordées aux deuxièmes extrémités de raccordement desdits capteurs, **caractérisé en ce que** :
ledit élément de conduite est sensiblement rigide et présente un diamètre interne D sensiblement égal à celui des portions de tubulure pour produire un écoulement laminaire dudit liquide ;
**en ce que** chaque moyen de mesure de pression comprend en outre :
- des moyens formant tubulure présentant un alésage axial dont la face interne de la paroi constitue sensiblement une solution de continuité de la tubulure et s'étendant entre les deux extrémités de raccordement, lesdits moyens comportant un orifice traversant la paroi et débouchant dans la face interne, et
- un capteur de pression pour mesurer la pression dudit liquide dans la tubulure, ledit capteur présentant une surface soumise à la pression dudit liquide, au moins une partie dudit capteur étant montée de manière étanche dans ledit orifice, de telle manière que ladite surface sensible constitue sensiblement une solution de continuité de la face interne de la paroi des moyens formant tubulure.
et **en ce que** ledit dispositif comprend en outre des moyens de traitement des deux pressions mesurées pour en déduire un débit.

2. Dispositif selon la revendication 1, **caractérisé en ce que** le volume mort limité par la surface sensible du capteur et par la surface sur laquelle est inscrite la face interne de la paroi des moyens formant tubulure est inférieur à 10 mm³.

3. Dispositif de mesure selon l'une quelconque des revendications 1 et 2, **caractérisé en ce que** le diamètre interne D de l'élément de conduite est compris entre 1 et 3 mm.

4. Dispositif de mesure selon l'une quelconque des revendications 1 à 3, **caractérisé en ce que** la longueur de l'élément de conduite est comprise entre 1,5 et 2,5 m.

5. Dispositif selon la revendication 4, **caractérisé en ce que** la longueur de l'élément de conduite (16) est égale à 2 m.

6. Dispositif de mesure selon l'une quelconque des revendications 1 à 5, **caractérisé en ce que** ledit élément de conduite est réalisé en silicone et présente une épaisseur de paroi au moins égale à 0,8 mm.

## Patentansprüche

1. Vorrichtung zum Messen des Durchflusses einer Flüssigkeit, die in einer Leitung zirkuliert und dem menschlichen Körper entzogen oder zugeführt wird, mit zwei Druckmessmitteln, wobei jedes Druckmessmittel ein erstes Ende umfasst, das mit einem ersten bzw. zweiten Stück dieser Leitung verbunden ist, sowie ein zweites Verbindungsendstück und ein Rohrleitungsteil, dessen Enden jeweils mit den zweiten Verbindungsendstücken dieser Messfühler verbunden sind,
**dadurch gekennzeichnet, dass** das Rohrleitungsteil nahezu steif ist und einen Innendurchmesser D aufweist, der im wesentlichen dem der Leitungsstücke entspricht, um eine laminare Strömung der Flüssigkeit zu bewirken;
dass jedes Druckmessmittel zudem umfasst:
- leitungsbildende Mittel, die eine axiale Bohrung aufweisen, deren Wandinnenseite im wesentlichen eine Unterbrechung der Kontinuität der Leitung bildet, die sich zwischen den beiden Verbindungsendstücken erstreckt, wobei diese Mittel eine Öffnung aufweisen, die die Wand durchquert und in die Innenseite mündet, und
- einen Druckmessfühler zum Messen des Drucks der Flüssigkeit in der Leitung, wobei der Messfühler eine Seite aufweist, die dem Druck der Flüssigkeit ausgesetzt ist, wobei wenigstens ein Teil des Messfühlers in dichter Weise in der Öffnung angebracht ist, so dass die empfindliche Seite im wesentlichen eine Unterbrechung der Kontinuität der Wandinnenseite der leitungsbildenden Mittel bildet.
und dass diese Vorrichtung zudem Mittel zum Verarbeiten der beiden gemessenen Drücke umfasst, um hieraus eine Durchflussleistung abzuleiten.

2. Vorrichtung nach Anspruch 1,
**dadurch gekennzeichnet, dass** das Totraumvolumen, das durch die empfindliche Seite des Messfühlers und die Seite begrenzt wird, an der die Wandinnenseite der leitungsbildenden Mittel liegt, kleiner als 10 mm³ ist.

3. Messvorrichtung nach einem der Ansprüche 1 bis 2,
**dadurch gekennzeichnet, dass** der Innendurchmesser D des Rohrleitungsteils 1 bis 3 mm beträgt.

4. Messvorrichtung nach einem der Ansprüche 1 bis 3,
**dadurch gekennzeichnet, dass** die Länge des Rohrleitungsteils 1,5 bis 2,5 m beträgt.

5. Vorrichtung nach Anspruch 4,
**dadurch gekennzeichnet, dass** die Länge des Rohrleitungsteils (16) 2 m beträgt.

6. Messvorrichtung nach einem der Ansprüche 1 bis 5,
**dadurch gekennzeichnet, dass** dieses Rohrleitungsteil aus Silikon ausgeführt ist und eine Wanddicke von mindestens 0,8 mm aufweist.

## Claims

1. Device for measuring the flow rate of a liquid circulating in a pipe and extracted from or introduced in the human body comprising two pressure measuring means, each pressure measuring means including a first end connected respectively to a first and a second portion of said pipe, and a second end for connection and a conduit element of which the ends are respectively connected to the second connection ends of said sensors, the device being **characterised in that**:
said conduit element is substantially rigid and has an internal diameter D substantially equal to that of the pipe portions to produce a laminar flow of said liquid;
**in that** each pressure measuring means further comprises:
- pipe-forming means presenting an axial bore of which the inner surface of the wall substantially provides continuity to the pipe and extending between the two connection ends, said means comprising an orifice passing through the wall and emerging in the inner surface, and
- a pressure sensor for measuring said liquid pressure in the pipe, said sensor having a surface subjected to the pressure of said liquid, at least a part of said sensor being mounted sealed in said orifice, such that said sensitive surface substantially provides continuity to the inner wall surface of the pipe-forming means.
and **in that** said device further comprises means for processing the two pressures measured in order to deduce a flow rate therefrom,

2. Device according to Claim 1, **characterised in that** the dead volume limited by the sensitive surface of the sensor and by the surface over which the internal face of the wall of the pipe-forming means is inscribed, is less than 10 mm³.

3. Measuring device according to Claim 1 or 2, **characterised in that** the internal diameter D of the conduit element is included between 1 mm and 3 mm.

4. Measuring device according to any one of Claims 1 to 3, **characterised in that** the length of the conduit element is included between 1.5 m and 2.5 m.

5. Measuring device according to Claim 4, **characterised in that** the length of the conduit element (16) is equal to 2 m.

6. Measuring device according to any one of Claims 1 to 5, **characterised in that** said conduit element is made of silicone and presents a wall thickness at least equal to 0.8 mm.
